# EUROPÄISCHE PATENTANMELDUNG

(11) **EP 1 086 939 A1**
(43) Veröffentlichungstag der Anmeldung: **28.03.2001**
(21) Anmeldenummer: 00120921.2
(22) Anmeldetag: 26.09.2000
(51) Int. Cl.: C07C 7/20, C09K 15/08, C09K 15/20

(54) **Inhibitorzusammensetzung**

(30) Priorität: 27.09.1999 DE 19946136
(71) Anmelder: BASF AKTIENGESELLSCHAFT, 67056 Ludwigshafen (DE)
(72) Erfinder: Sutoris, Heinz Friedrich, 67551 Worms (DE); Wagenblast, Gerhard, 67157 Wachenheim (DE); Schliephake, Volker, 67105 Schifferstadt (DE); Schröder, Jürgen, 67071 Ludwigshafen (DE); Keller, Harlad, 67069 Ludwigshafen (DE); Nestler, Gerhard, 67061 Ludwigshafen (DE)
(74) Vertreter: Kinzebach, Werner, Dr.

(57) **Zusammenfassung**

Beschrieben wird eine phosphorfreie Inhibitorzusammensetzung, umfassend
a) wenigstens ein N-Oxyl-Radikal und
b) eine Verbindung der allgemeinen Formel (X) 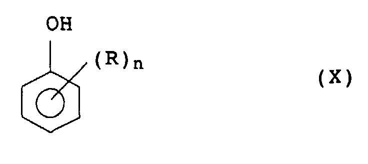 worin
   - n: für eine ganze Zahl von 0 bis 3 steht und
   - R: für C₁-C₄-Alkyl, C₂-C₄-Alkenyl oder C₇-C₁₀-Aralkyl steht.

Die Inhibitorzusammensetzung wird zur Verhütung unerwünschter Polymerisation ethylenisch ungesättigter Verbindungen verwendet.

## Beschreibung

Die vorliegende Erfindung betrifft eine phosphorfreie Inhibitorzusammensetzung, ihre Verwendung zur Verhütung unerwünschter Polymerisation von ethylenisch ungesättigten Verbindungen und ein Stoffgemisch, das wenigstens eine ethylenisch ungesättigte Verbindung und die Inhibitorzusammensetzung enthält.

Chemische Verbindungen, die eine oder mehrere ethylenisch ungesättigte Gruppen aufweisen, haben eine ausgeprägte Neigung zur radikalischen Polymerisation. Solche Verbindungen werden als Monomere zur gezielten Herstellung von Polymerisaten, z. B. durch radikalische Polymerisation, verwendet. Gleichzeitig ist die ausgeprägte Neigung zur radikalischen Polymerisation aber insofern von Nachteil, als es sowohl bei der Lagerung als auch bei der chemischen und/oder physikalischen Bearbeitung, z. B. durch Destillation oder Rektifikation, der ethylenisch ungesättigten Verbindungen, insbesondere unter der Einwirkung von Wärme und/oder Licht, zur unerwünschten, spontanen radikalischen Polymerisation kommen kann. Derartige unkontrollierte radikalische Polymerisationen bergen ein erhebliches Gefahrenpotential und verlaufen häufig explosionsartig. Bei der Destillation von ethylenisch ungesättigte Verbindungen enthaltenden Gemischen kann sich beispielsweise unkontrolliert gebildetes Polymerisat auf der Oberfläche des Verdampfers niederschlagen - dort ist die Neigung zur Polymerisatbildung infolge der hohen Temperaturen erhöht - und dadurch den Wärmeübergang in unerwünschter Weise mindern. Gebildetes Polymerisat kann aber auch die Einbauten in Rektifikationskolonnen verstopfen, was unerwünschte Druckverluste verursacht. Letztlich muss der Rektifikationsprozess unterbrochen werden, um das gebildete Polymerisat zu entfernen.

Es ist daher üblich, ethylenisch ungesättigten Verbindungen beziehungsweise Gemischen, die solche Verbindungen enthalten, sowohl bei der Lagerung als auch bei der chemischen und/oder physikalischen Bearbeitung Substanzen zuzusetzen, die eine spontane Polymerisation unterbinden beziehungsweise verlangsamen. Sie werden im Allgemeinen als Inhibitoren beziehungsweise Retarder bezeichnet und unter dem Oberbegriff "Stabilisatoren" zusammengefasst.

Die US-A-2,105,284 schlägt Phenole als Polymerisationsinhibitoren für Isobutenal und Methacrylsäure vor.

Die DE-A-24 20 968 beschreibt die Stabilisierung von Acrylsäure durch Phenole, wie 2,6-Di-t-butyl-4-methylphenol und 4-Methoxyphenol.

Die DD-0 272 067 beschreibt die Umesterung von (Meth)acrylaten mit mehrwertigen Alkoholen, wobei das Reaktionsgemisch mit 2,6-Di-t-butyl-4-methylphenol stabilisiert wird.

Die DE 38 43 843 und die DE 38 43 930 beschreiben die Umsetzung mehrwertiger Alkohole mit Acrylsäure unter Stabilisierung mit Tocopherol und Luft.

Die JP-07/025.907 beschreibt die Stabilisierung bestimmter (Meth)acrylate, wie 3-Methacryloyloxypropyltrichlorsilan, mit 2,6-Di-t-butyl-4-hydroxymethylphenol.

Die JP-10/175.919 beschreibt die Verwendung einer Stabilisatorkombination aus einem sterisch gehinderten Phenol, wie 2,6-Di-t-butyl-4-methylphenol, und Luft bei der Umsetzung von Tetrahydrobenzylalkohol mit (Meth) acrylsäure.

Die DE 16 18 141 schlägt die Verwendung von N-Oxylen, wie 2,2,6,6-Tetramethylpiperidin-1-oxyl, zur Stabilisierung von Acrylsäure vor.

Die EP-0 178 168 schlägt die Verwendung von 2,2,6,6,-Tetramethyl-4-oxypiperidin-1-oxyl als Stabilisator bei der Destillation von Methacrylsäure vor.

Die US-A-5,322,960 beschreibt ein Verfahren zur Stabilisierung von (Meth)acrylsäure und deren Estern gegen Polymerisation während Herstellung und Lagerung durch eine Kombination einer N-Oxyl-Verbindung, eines mehrwertigen Phenols und einer Phenothiazinverbindung.

Die US-A-5,728,872 offenbart eine stabilisierte Acrylsäure-Zusammensetzung, die neben Acrylsäure ein stabiles Nitroxylradikal und ein diheterosubstituiertes Benzolderivat, wie Hydrochinonmonomethylether, enthält.

Die nicht vorveröffentlichte DE 199 38 841.5 betrifft eine Inhibitorkomposition aus einem N-Oxyl-Radikal, einem Phenol und einer Phosphor in der Oxidationszahl +3 enthaltenden Verbindung. Die Inhibitorkomposition dient zur Stabilisierung radikalisch polymerisierbarer Verbindungen.

Es ist nun gefunden worden, dass N-Oxylradikale in Verbindung mit einwertigen Phenolen eine synergistische Wirkung bei der Stabilisierung ethylenisch ungesättigter Verbindungen gegen unerwünschte Polymerisation zeigen.

Demzufolge betrifft die Erfindung eine phosphorfreie Inhibitorzusammensetzung, die umfasst
a) wenigstens ein N-Oxyl-Radikal und
b) eine Verbindung der allgemeinen Formel (X) 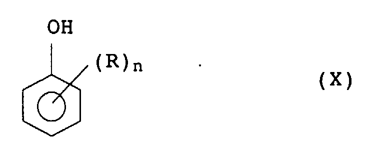 worin
   - n: für eine ganze Zahl von 0 bis 3 steht und
   - R: für C₁-C₄-Alkyl, C₂-C₄-Alkenyl oder C₇-C₁₀-Aralkyl steht.

Die Erfindung betrifft außerdem die Verwendung der Inhibitorzusammensetzung zur Verhütung unerwünschter Polymerisation von ethylenisch ungesättigten Verbindungen, z. B. während der Herstellung, Lagerung, dem Transport oder der chemischen und/oder physikalischen Bearbeitung.

Die Erfindung betrifft außerdem ein Verfahren zur Stabilisierung von Materialien, die wenigstens eine ethylenisch ungesättigte Verbindung umfassen, gegen unerwünschte Polymerisation, indem man zu den zu stabilisierenden Materialien eine oben beschriebene Inhibitorzusammensetzung gibt. Die Komponenten der Zusammensetzung können gleichzeitig oder nacheinander zugegeben werden. Die zu stabilisierenden Materialien können ausschließlich aus einer oder mehreren ethylenisch ungesättigten Verbindungen bestehen oder diese in einer Konzentration enthalten, dass bei spontaner Initiierung eine radikalische Polymerisation ablaufen kann. Vorzugsweise werden neben der erfindungsgemäßen Inhibitorzusammensetzung keine weiteren polymerisationsinhibierenden Zusätze verwendet.

Die Erfindung betrifft außerdem ein Stoffgemisch, das wenigstens eine ethylenisch ungesättigte Verbindung und eine zu Verhütung unerwünschter Polymerisation wirksame Menge der oben beschriebenen Inhibitorzusammensetzung enthält.

Sämtliche Ausführungen in der vorliegenden Beschreibung, die mit Bezug auf die Inhibitorzusammensetzung erfolgen, gelten entsprechend für das erfindungsgemäße Verfahren, die erfindungsgemäße Verwendung und das erfindungsgemäße Stoffgemisch.

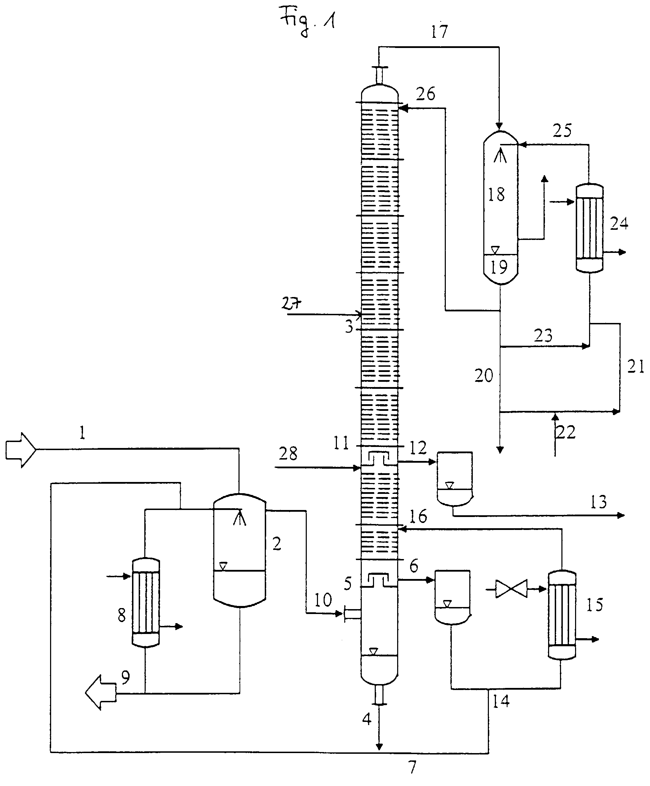

Die beiliegende Fig. 1 zeigt schematisch eine Vorrichtung zur Isolierung von Acrylsäure aus einem durch heterogen katalysierte Gasphasenoxidation erhaltenen Gasgemisch, wobei die erfindungsgemäße Inhibitorzusammensetzung verwendet wird.

Die erfindungsgemäße Inhibitorzusammensetzung ist phosphorfrei, d. h. sie enthält keine phosphorhaltigen Verbindungen, wie Phosphite oder Phosphonite.

Die erfindungsgemäße Inhibitorzusammensetzung ist vorzugsweise schwefeifrei, d. h. sie enthält vorzugsweise keine schwefelhaltigen Verbindungen, wie insbesondere Phenothiazinverbindungen.

Die Abwesenheit von P- und vorzugsweise S-Heteroatomen ist vorteilhaft, da Destillationsrückstände, in denen sich die erfindungsgemäße Inhibitorzusammensetzung bei bzw. nach der Verwendung anreichert, vielfach verbrannt werden. Die Anwesenheit nicht systemimmanenter Heteroatome kann dabei zu unerwünschten Emissionen führen.

Bei den N-Oxyl-Radikalen (Nitroxylradikalen) handelt es sich um sogenannte stabile bzw. persistente freie Radikale, die in der Regel als Reinsubstanz herstellbar und über lange Zeiträume unzersetzt gelagert werden können. Sie können ein oder mehrere Radikalzentren im Molekül aufweisen. Sie leiten sich im Allgemeinen von einem sekundären Amin ab, worin sämtliche Substituenten in α-Position zum Stickstoffatom von Wasserstoff verschieden sind.

Geeignete N-Oxyl-Radikale sind z. B. jene der allgemeinen Formel I 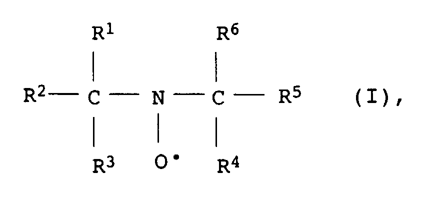 mit
- R¹, R², R⁵ und R⁶: = dieselben oder verschiedene gerad- oder verzweigtkettige, gegebenenfalls substituierte Alkylgruppen und
- R³ und R⁴: = dieselben oder verschiedene gerad- oder verzweigtkettige, gegebenenfalls substituierte Alkylgruppen oder
- R³CNCR⁴: = eine gegebenenfalls substituierte, zyklische Struktur.

Als erfindungsgemäß geeignete Verbindungen I kommen insbesondere jene in Betracht, die in der EP-A 135 280, der DE-A 19651307, der US-A 5,322,912, der US-A 5,412,047, der US-A 4,581,429, der DE-A 16 18 141, CN-A 1052847, US-A 4,670,131, US-A 5,322,960 sowie der DE-A 19602539 genannt sind.

Beispiele dafür sind jene stabilen N-Oxyl-Radikale der allgemeinen Formel I, bei welchen R¹, R², R⁵ und R⁶ für (gleiche oder verschiedene) C₁- bis C₄-Alkylgruppen wie Methyl-, Ethyl-, n-Propyl-,iso-Propyl-, n-Butyl-, iso-Butyl- oder tert.-Butyl-, lineares oder verzweigtes Pentyl-, Phenyl- oder substituierte Gruppen hiervon und R³ und R⁴ für (gleiche oder verschiedene) C₁- bis C₄-Alkylgruppen wie Methyl-, Ethyl-, n-Propyl-, iso-Propyl-, n-Butyl-, iso-Butyl- oder tert.-Butyl-, lineares oder verzweigtes Pentyl-, substituierte Gruppen hiervon oder gemeinsam mit CNC die die zyklische Struktur 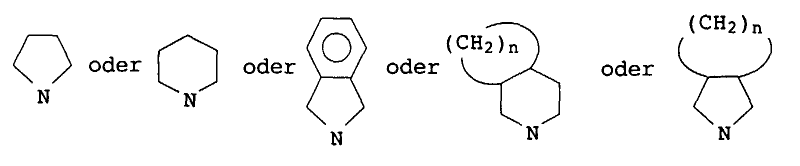 mit n gleich einer ganzen Zahl von 1 bis 10 (häufig 1 bis 6), einschließlich substituierter derartiger zyklischer Strukturen, stehen.

Die N-Oxyl-Radikale I lassen sich aus den entsprechenden sekundären Aminen durch Oxidation, z. B. mit Wasserstoffperoxid, herstellen.

Zu den erfindungsgemäß geeigneten N-Oxyl-Radikalen zählen insbesondere Piperidin-, Pyrazolidin- oder Pyrrolidin-N-Oxylradikale. Sie weisen z. B. eine der nachstehenden allgemeinen Formeln II bis IX auf: 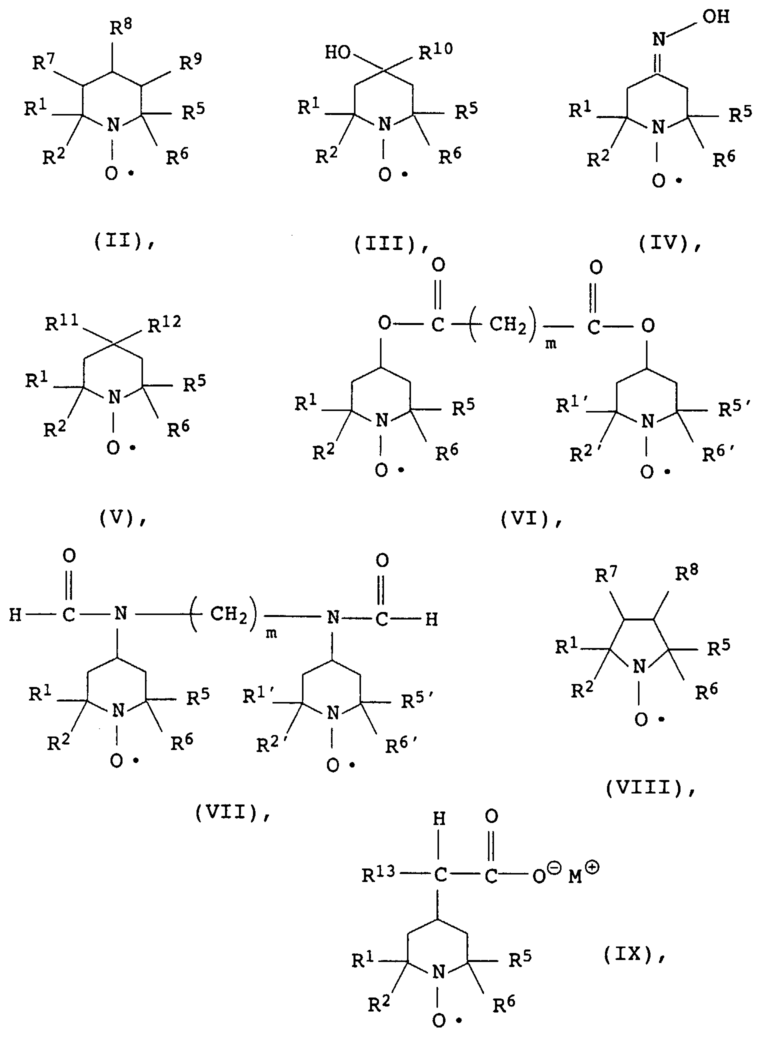 mit
- m =: 2 bis 10,
- R⁷, R⁸, R⁹ =: unabhängig voneinander 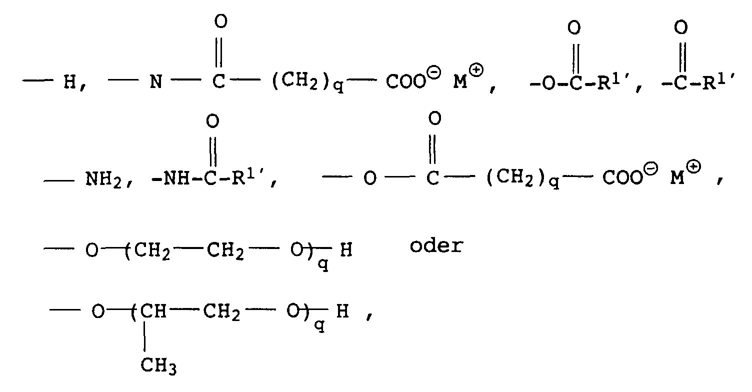
- M^{⊕} =: ein Wasserstoff- oder ein Alkalimetallion,
- q =: eine ganze Zahl von 1 bis 10,
- R¹', R²', R⁵', R⁶': = unabhängig voneinander und unabhängig von R¹, R², R⁵, R⁶ dieselben Gruppen wie R¹,
- R¹⁰ =: C₁- bis C₄-Alkyl, -CH=CH₂, -C≡CH, -CN, 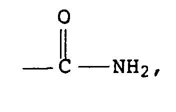 -COO^{⊖} M^{⊕}, -COOCH₃ oder -COOC₂H₅,
- R¹¹ =: ein organischer Rest, der wenigstens eine primäre, sekundäre (z. B. -NHR¹) oder tertiäre Aminogruppe (z. B. -NR¹R²) oder wenigstens eine Ammoniumgruppe -N^{⊕}R¹⁴R¹⁵R¹⁶X^{⊖} aufweist, mit X^{⊖} = F^{⊖}, Cl^{⊖}, Br^{⊖} und R¹⁴, R¹⁵, R¹⁶ voneinander unabhängige organische Reste (z. B. unabhängig voneinander und unabhängig von R¹ dieselben Gruppen wie R¹),
- R¹² =: unabhängig von R¹¹ dieselben Gruppen wie R¹¹ oder -H, -OH, C₁- bis C₄-Alkyl, -COO^{⊖}M^{⊕}, -C≡CH, 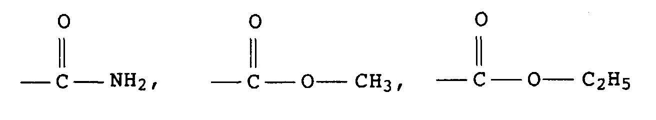 oder hydroxysubstituiertes C₁- bis C₄-Alkyl (z. B. hydroxyethyl oder hydroxypropyl) oder
- R¹¹, R¹² =: gemeinsam den Sauerstoff einer Carbonylgruppe und
- R¹³ =: ―H, ―CH₃ oder

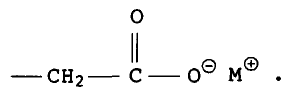

Vorzugsweise ist R¹ = R² = R⁵ = R⁶ = R^{1'} = R^{2'} = R^{5'} = R^{6'} = -CH₃.

Als beispielhafte Vertreter erfindungsgemäß geeigneter N-Oxyl-Radikale seien 1-Oxyl-2,2,6,6-tetramethylpiperidin, 1-Oxyl-2,2,6,6-tetramethylpiperidin-4-ol, 1-Oxyl-2,2,6,6-tetramethyl-4-methoxypiperidin, 1-Oxyl-2,2,6,6-tetramethyl-4-ethoxypiperidin, 1-Oxyl-2,2,6,6-tetramethyl-4-trimethylsiloxypiperidin, 1-Oxyl-2,2,6,6-tetramethylpiperidin-4-on, 1-Oxyl-2,2,6,6-tetramethylpiperidin4-yl-acetat, 1-Oxyl-2,2,6,6-tetramethylpiperidin-4-yl-2-ethylhexanoat, 1-Oxyl-2,2,6,6-tetramethylpiperidin-4-yl-stearat, 1-Oxyl-2,2,6,6-tetramethylpiperidin-4-yl-benzoat, 1-Oxyl-2,2,6,6-tetramethylpiperidin-4-yl-(4-tert-butyl)benzoat, Bis(1-oxyl-2,2,6,6-tetramethylpiperidin-4-yl)-succinat, Bis(1-oxyl-2,2,6,6-tetramethylpiperidin-4-yl)-adipat, Bis(1-oxyl-2,2,6,6-tetramethylpiperidin-4-yl)-sebacat, Bis(1-oxyl-2,2,6,6-tetramethylpiperidin-4-yl)-n-butylmalonat, Bis(1-oxyl-2,2,6,6-tetramethylpiperidin-4-yl)-phthalat, Bis(1-oxyl-2,2,6,6-tetramethylpiperidin-4-yl)-isophthalat, Bis(1-oxyl-2,2,6,6-tetramethylpiperidin-4-yl)-terephthalat, Bis(1-oxyl-2,2,6,6-tetramethylpiperidin-4-yl)-hexahydroterephthalat, N,N'-Bis(1-oxyl-2,2,6,6-tetramethylpiperidin4-yl)-adipinamid, N-(1-Oxyl-2,2,6,6-tetramethylpiperidin-4-yl)-caprolactam, N-(1-Oxyl-2,2,6,6-tetramethylpiperidin-4-yl)-dodecylsuccinimid, 2,4,6-Tris-[N-butyl-N-(1-oxyl-2,2,6,6-tetramethylpiperidin-4-yl]-s-triazin, N,N'-Bis(1-oxyl-2,2,6,6-tetramethylpiperidin-4-yl)-N,N' -bis-formyl-1,6-diaminohexan, 4,4'- Ethylenbis(1-oxyl-2,2,6,6-tetramethylpiperazin-3-on) und 1-Oxyl-2,2,6,6-tetramethyl-1,2,3,6-tetrahydropyridin genannt.

Die Verbindungen (VI) und (VII) können gemäß US-A 4665185 (z. B. Bsp. 7) sowie DE-A 19510184 erhalten werden.

Weitere geeignete beispielhafte Vertreter sind (in den Strukturformeln sind die Heteroatome mit H-Atomen (nicht eingezeichnet) abgesättigt):

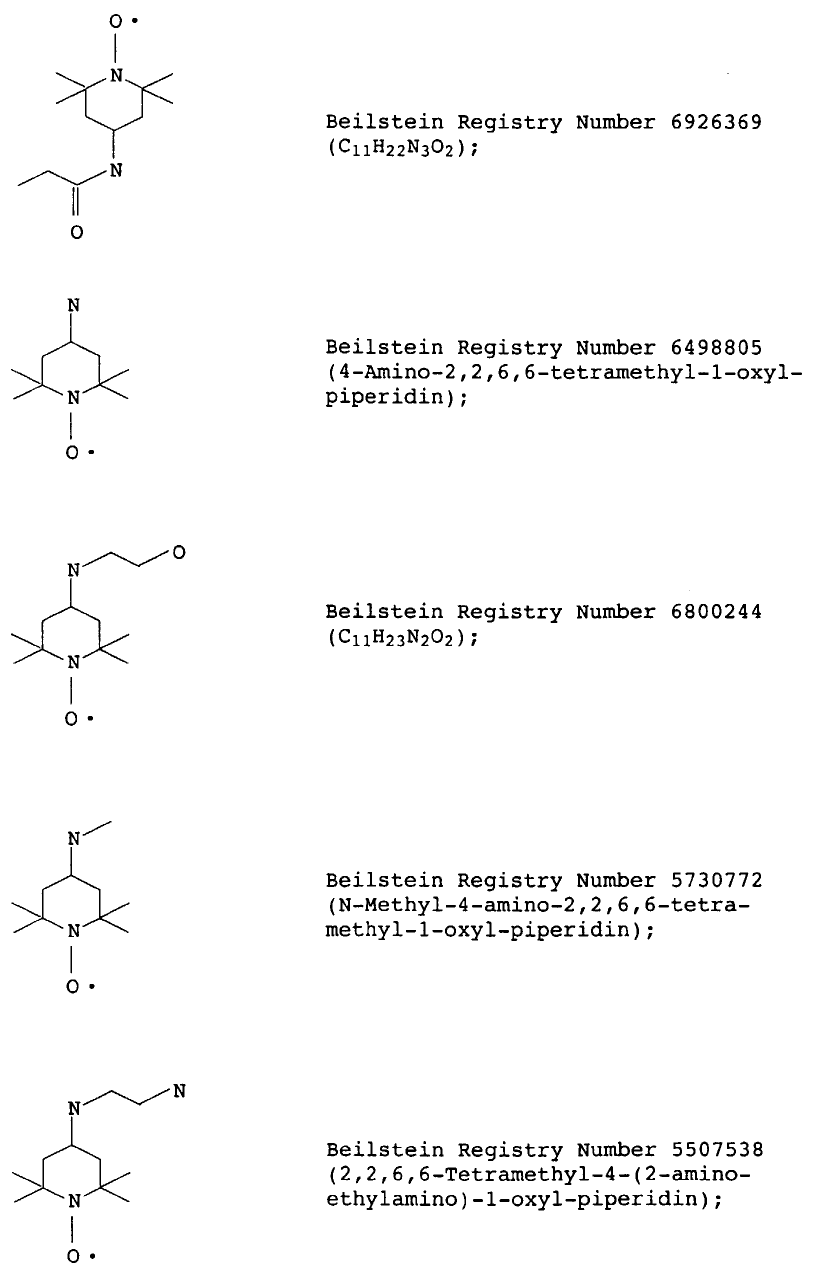

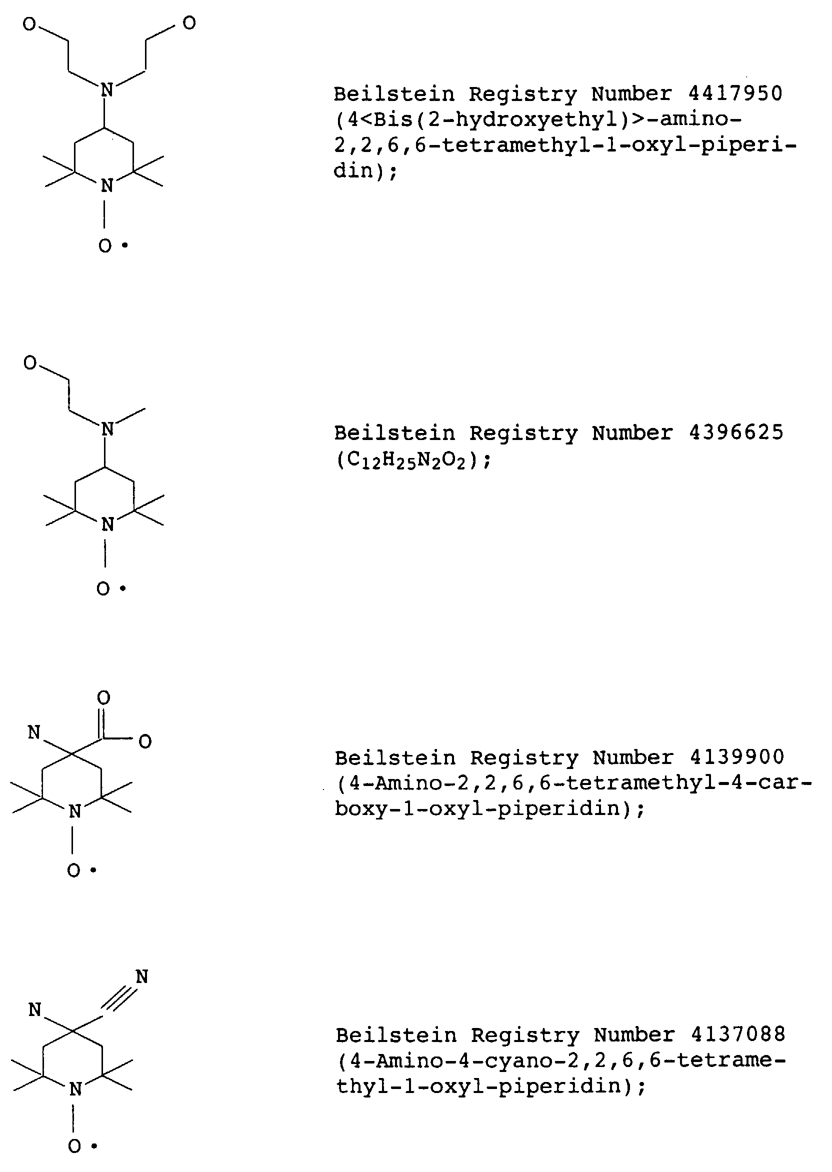

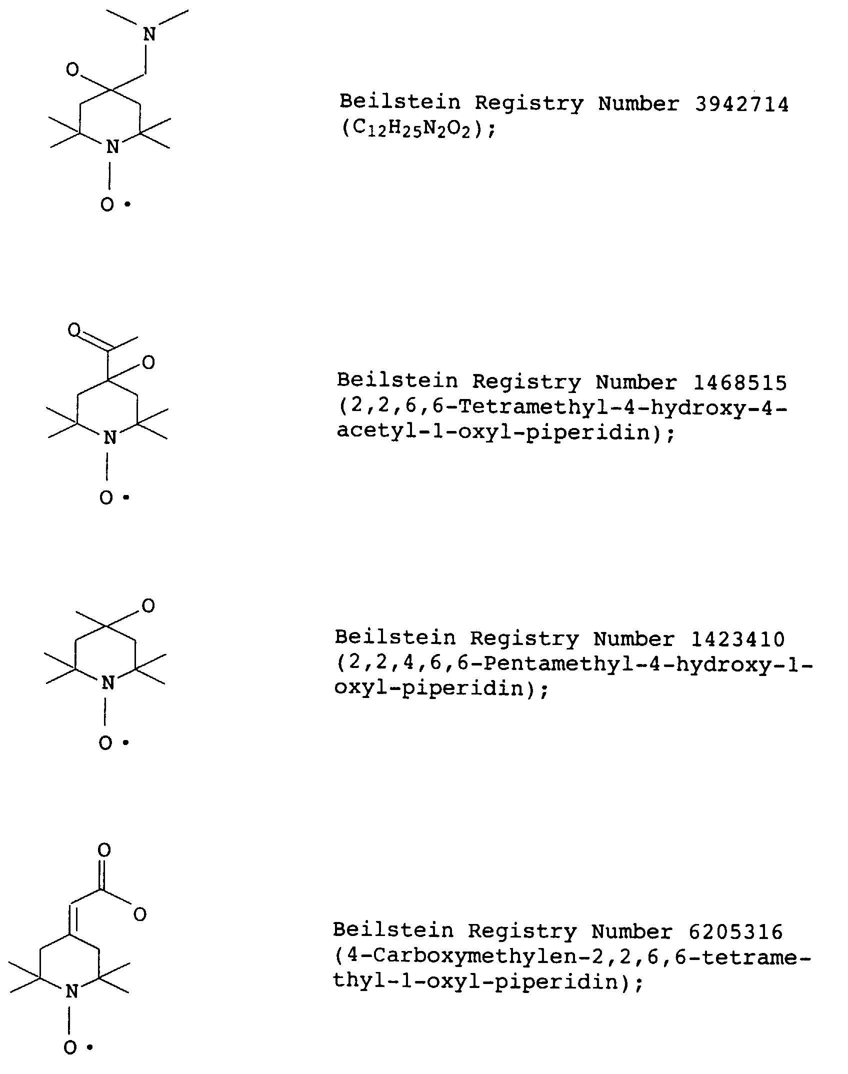

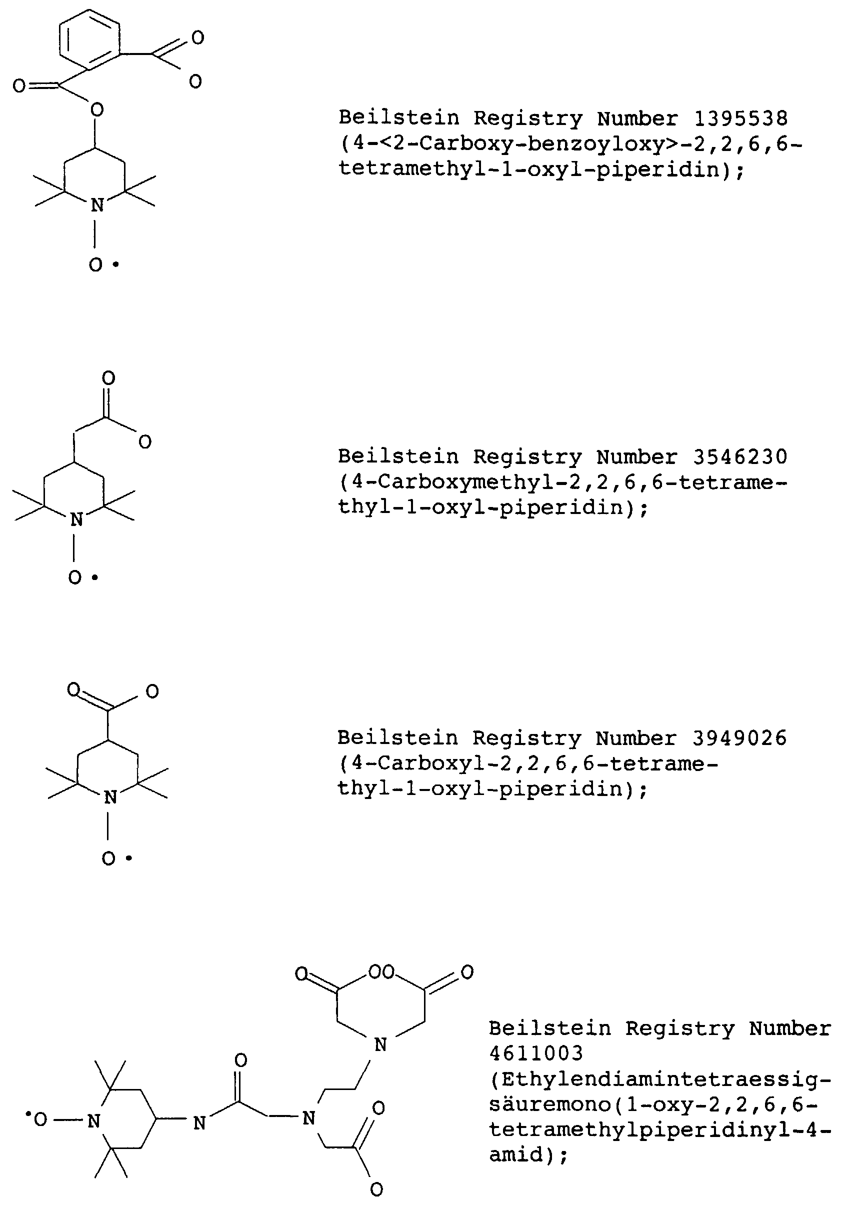

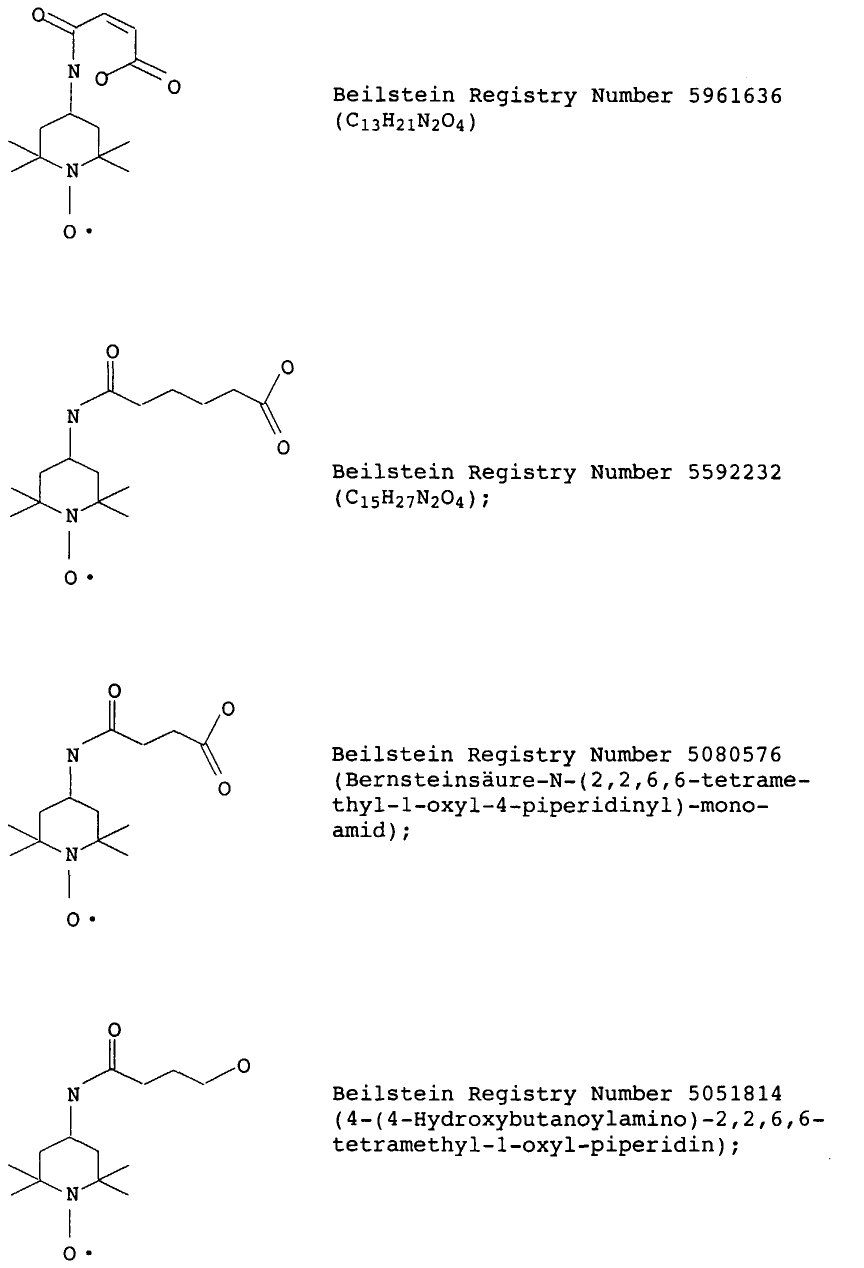

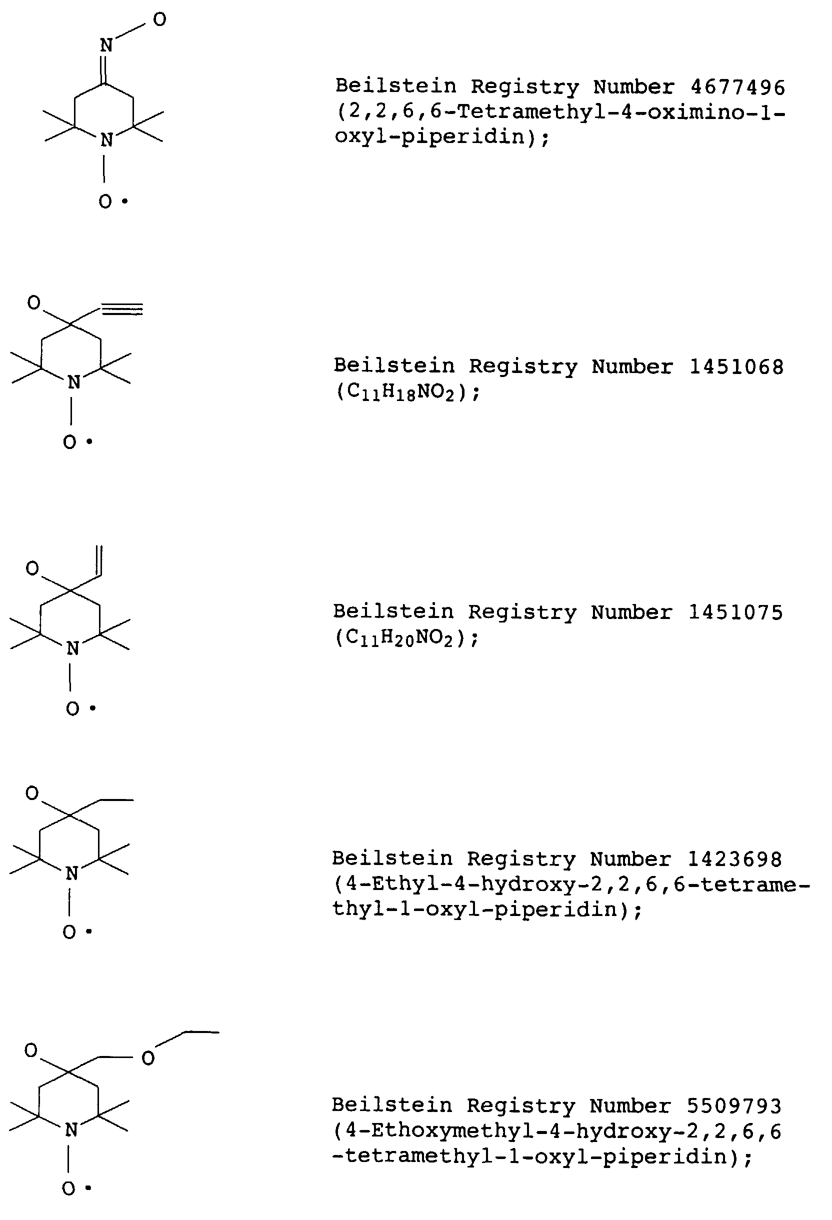

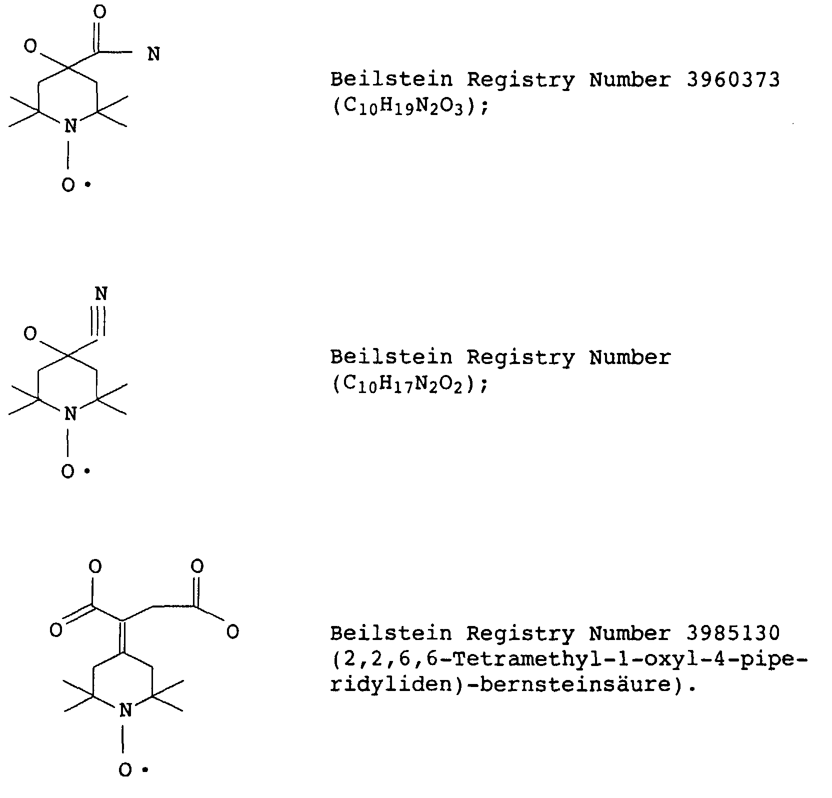

Weitere erfindungsgemäß geeignete N-Oxyl-Radikale sind diejenigen, die in der WO 98/44008, der WO 97/46593 und der DE-A 19743396 offenbart sind.

Die erfindungsgemäße Inhibitorzusammensetzung umfasst ferner eine Verbindung der allgemeinen Formel (X)

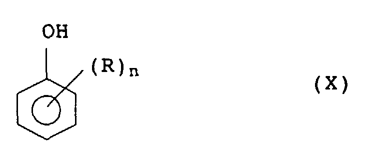

In der Formel (X) steht n für eine ganze Zahl von 0 bis 3, vorzugsweise für 0, 1 oder 2. R steht für C₁-C₄-Alkyl, vorzugsweise Methyl, Ethyl, n-Propyl, Isopropyl, n-Butyl, Isobutyl oder t-Butyl, C₂-C₄-Alkenyl, insbesondere Allyl, oder C₇-C₁₀-Aralkyl, insbesondere Benzyl. Methyl und t-Butyl sind besonders bevorzugt. Vorzugsweise ist die Verbindung der Formel (X) ausgewählt unter 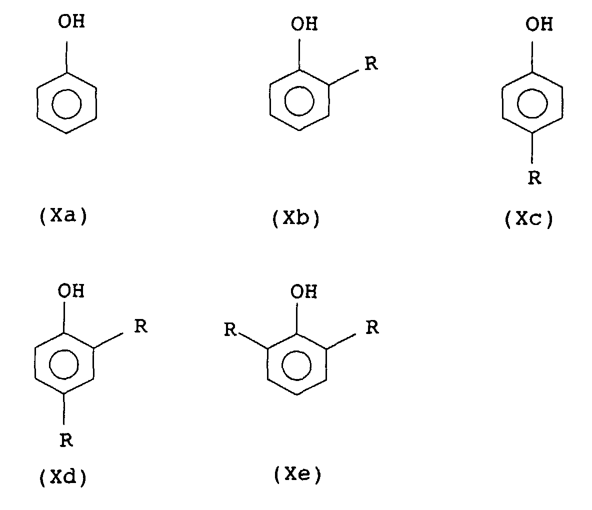 worin R die angegebene Bedeutung hat.

Vorzugsweise ist die Verbindung der Formel (X) ausgewählt unter Phenol, 2-Methylphenol, 4-Methylphenol, 2-t-Butylphenol, 4-t-Butylphenol, 2-t-Butyl-4-methylphenol, 2,4-Dimethylphenol, 2,6-Dimethylphenol, 2,4-Di-t-butylphenol, 2-Benzylphenol oder 2-Allylphenol oder Mischungen davon.

In der Regel setzt man dem zu stabilisierenden Material 1 bis 2000 Gew.-ppm, häufig 50 bis 500 Gew.-ppm (bezogen auf die enthaltene Menge an ethylenisch ungesättigten Verbindungen) der erfindungsgemäßen Inhibitorverbindung zu.

Das Gewichtsverhältnis von N-Oxyl-Radikal und Verbindung der Formel (X) beträgt im Allgemeinen 2:1 bis 1:100, vorzugsweise 1:1 bis 1:50, insbesondere 1:2 bis 1:25, besonders bevorzugt 1:3 bis 1:10.

Selbstverständlich kann die erfindungsgemäße Inhibitorzusammensetzung noch weitere eine radikalische Polymerisation inhibierende Bestandteile umfassen.

Beispiele für solche sonstigen radikalischen Polymerisationsinhibitoren sind organische Nitrosoverbindungen wie N-Nitrosoarylamine oder die Nitrosogruppe unmittelbar an ein Kohlenstoffatom eines aromatischen Kerns gebunden aufweisende Nitrosoverbindungen. Beispielhaft genannt seien Nitrosophenole wie 4-Nitrosophenol, aber auch Nitrosobenzol.

Günstig ist, dass das erfindungsgemäße Inhibitorgemisch seine Wirksamkeit auch in Gegenwart von molekularem Sauerstoff entfaltet.

Als wenigstens eine ethylenisch ungesättigte Gruppe aufweisende chemische Verbindungen, die erfindungsgemäß stabilisiert werden können, kommen u. a. Verbindungen in Betracht wie die Olefine, z. B. Isobuten, Ethylen, Propylen, vinylaromatische Monomere wie Styrol, α-Methylstyrol, o-Chlorstyrol oder Vinyltoluole, C₄-C₈-konjugierte Diene, wie Butadien oder Isopren, Ester aus Vinylalkohol und 1 bis 18 C-Atome aufweisenden Monocarbonsäuren wie Vinylacetat, Vinylpropionat, Vinyl-n-butyrat, Vinyllaurat und Vinylstearat.

Insbesondere ist die erfindungsgemäße Stabilisierung aber im Fall von 3 bis 6 C-Atome aufweisenden α,β-monoethylenisch ungesättigten Mono- und Dicarbonsäuren, wie insbesondere Acrylsäure, Methacrylsäure, Maleinsäure, Fumarsäure und Itaconsäure, die Ester aus den vorgenannten Carbonsäuren und 1 bis 12, vielfach 1 bis 8 und häufig 1 bis 4 C-Atome aufweisenden Alkanolen, wie insbesondere Acrylsäure- und Methacrylsäuremethyl-, -ethyl-, -n-butyl-, -isobutyl-, -tert.-butyl- und -2-ethylhexylester, Maleinsäuredimethylester oder Maleinsäure-di-n-butylester, geeignet. Die erfindungsgemäße Zusammensetzung eignet sich aber auch zur Stabilisierung der Vorläuferaldehyde, Nitrile und Amide der vorgenannten 3 bis 6 C-Atome aufweisenden α,β-monoethylenisch ungesättigten Mono- und Dicarbonsäuren wie z. B. Acrolein, Methacrolein, Acrylnitril, Methacrylnitril, Acrylamid und Methacrylamid. Sie ist aber auch anwendbar im Fall von Monomeren wie Vinylsulfonsäure und N-Vinylpyrrolidon.

Die erfindungsgemäße Zusammensetzung ist sowohl zur Lagerstabilisierung als auch zur Prozessstabilisierung bei der Herstellung, Reinigung (z. B. Destillation) und chemischen Umsetzung von ethylenisch ungesättigten Verbindungen geeignet. Letzteres gilt auch für destillative Behandlungen bei Temperaturen von 50 bis 300 °C, häufig 50 bis 200 °C oder auch 50 bis 150 °C.

Insbesondere eignet sich die erfindungsgemäße Zusammensetzung zur Stabilisierung bei der destillativen (rektifikativen) Behandlung von (Meth)acrylsäureestern (insbesondere der vorgenannten beispielhaften Vertreter), bei deren destillativer oder rektifikativer Abtrennung aus Produktgemischen, wie sie als Ergebnis einer säurekatalysierten (homogen und/oder heterogen) Veresterung von (Meth)acrylsäure mit Alkoholen, insbesondere Alkanolen (insbesondere C₁- bis C₁₂- beziehungsweise C₁- bis C₈-Alkanolen) vor und/oder nach Abtrennung des Säurekatalysator vorliegen.

Sie eignet sich aber auch zur Stabilisierung von vorgenannte (Meth)acrylsäureester enthaltenden Gemischen, die weder den sauren Veresterungskatalysator noch Acryl- oder Methacrylsäure selbst enthalten. Solche (Meth)acrylsäureester enthaltenden Gemische bilden beispielsweise vorgenannte Veresterungsproduktgemische nach z. B. extraktiver (z. B. mittels Wasser und/oder wässriger Alkalilauge) und/oder rektifikativer Abtrennung des Säurekatalysators sowie nach entsprechender Abtrennung der überschüssigen (Meth)acrylsäure.

Die Stabilisierung eines einer Destillation (Rektifikation) unterworfenen (Meth)acrylsäureester enthaltenden Gemisches kann in einfacher Weise so erfolgen, dass man die erfindungsgemäße Inhibitorzusammensetzung dem Gemisch bereits vor der Destillation (Rektifikation) zugibt. Die Zugabe kann auch in den Zulauf zur Destillations(Rektifikations)kolonne erfolgen. Zur Stabilisierung kann zusätzlich oder ausschließlich auch eine Inhibitorzugabe auf den Kopf der Kolonne erfolgen.

Die Komponenten der erfindungsgemäßen Inhibitorzusammensetzung können zeitlich nacheinander, simultan oder auch bereits vorgemischt zugesetzt werden. Gleiches gilt auch für weitere Inhibitoren, falls das Inhibitorgemisch solche umfasst.

Die Destillations(Rektifikations)kolonnen können bei Anwendung der erfindungsgemäßen Inhibitorzusammensetzung von molekularem Sauerstoff oder einem Gemisch desselben mit einem Inertgas, z. B. Luft, durchströmt werden.

Die Zugabe der Komponenten der Inhibitorzusammensetzung kann an unterschiedlichen Zugabeorten erfolgen. So können z. B. Komponenten am Kopf der Rektifikationskolonne und andere Komponenten in den Sumpf und/oder den Zulauf der Rektifikationskolonne gegeben werden. Dies gilt z. B. für Rektifikationen, im Rahmen derer der (Meth)acrylsäureester über Kopf-, über Sumpf- und/oder über Seitenabzug abgetrennt wird. Auch kann es zweckmäßig sein, bei einer kontinuierlichen destillativen (rektifikativen) Abtrennung von (Meth)acrylsäureestern wenigstens eine Inhibitorkomponente lediglich von Zeit zu Zeit, d. h. periodisch wiederkehrend, zuzugeben (z. B. am Kolonnenkopf, im Sumpf und/oder im Zulauf).

Obige Ausführungen zur erfindungsgemäßen Stabilisierung bei der destillativen (rektifikativen) Abtrennung von (Meth)acrylsäureestern aus säurekatalysierten Veresterungsgemischen gelten analog auch bezüglich einer destillativen (rektifikativen) Abtrennung von (Meth)acrylsäure beziehungsweise (Meth)acrolein aus diese enthaltenden Gemischen.

Unter anderem ist (Meth)acrylsäure durch katalytische Gasphasenoxidation von Alkanen, Alkanolen, Alkenen oder Alkenalen erhältlich, die 3 beziehungsweise 4 C-Atome enthalten. Besonders vorteilhaft ist (Meth)acrylsäure z. B. durch katalytische Gasphasenoxidation von Propan, Propen, tert.-Butanol, iso-Buten, iso-Butan, iso-Butyraldehyd oder Methacrolein erhältlich. Als Ausgangsverbindungen sind aber auch solche denkbar, aus welchen sich die eigentliche C₃-/C₄-Ausgangsverbindung während der Gasphasenoxidation erst intermediär bildet. Beispielhaft genannt sei der Methylether des tert.-Butanols.

Dabei werden diese Ausgangsgase, in der Regel mit Inertgasen wie Stickstoff, CO, CO₂, gesättigten Kohlenwasserstoffen und/oder Wasserdampf verdünnt, im Gemisch mit Sauerstoff bei erhöhten Temperaturen (üblicherweise 200 bis 400°C) sowie gegebenenfalls erhöhtem Druck über übergangsmetallische (z. B. Mo, V, W und/oder Fe enthaltende) Mischoxidkatalysatoren geleitet und oxidativ in die (Meth) acrylsäure umgewandelt.

Bei der katalytischen Gasphasenoxidation wird in der Regel jedoch keine reine (Meth)acrylsäure sondern ein Reaktionsgasgemisch erhalten, das im Wesentlichen (Meth)acrylsäure, die inerten Verdünnungsgase und Nebenprodukte enthält, aus welchem die (Meth)acrylsäure abgetrennt werden muss. Neben vergleichsweise einfach zu entfernenden Nebenprodukten wie z. B. Essigsäure, enthält das Reaktionsgasgemisch häufig auch mit (Meth)acrylsäure eng verwandte und daher schwer abtrennbare niedere Aldehyde wie Formaldehyd, Acetaldehyd, Acrolein, Methacrolein, Propionaldehyd, n-Butyraldehyd, Benzaldehyd, Furfural und Crotonaldehyd sowie gegebenenfalls Maleinsäureanhydrid. Bezogen auf die im Reaktionsgasgemisch enthaltene Menge an (Meth)acrylsäure, beträgt die Gesamtmenge der Nebenkomponenten in der Regel ≤ 2 Gew.-%, meist ≥ 0,05 Gew.-%.

Die Abtrennung der (Meth)acrylsäure aus dem Reaktionsgasgemisch der katalytischen Gasphasenoxidation kann mit Vorteil durch Gegenstromabsorption mit einer hochsiedenden inerten Flüssigkeit und anschließender destillativer Aufarbeitung erfolgen (vgl. DE-A 21 36 396 und DE-A 43 08 087). Die Inhibitorzusammensetzung kann dabei direkt in den Gasstrom eingespritzt oder in der hochsiedenden Flüssigkeit vorgelegt werden. Alternativ kann man zunächst mit Wasser/wässriger Acrylsäure im Gegenstrom absorbieren und anschließend extraktiv oder azeotrop destillieren (vgl. EP-B 0 009 545, US 5,154,800, DE-A 34 29 391 und DE-A 21 21 123).

Als hochsiedende inerte hydrophobe organische Flüssigkeit kommen dabei u. a. Diphenyl, Diphenylether oder Dimethylphthalat sowie Gemische davon in Betracht.

Das Verfahren kann im Wesentlichen so durchgeführt werden, dass man das Reaktionsgasgemisch in einer konventionellen Absorptionskolonne zu der absteigenden Absorptionsflüssigkeit im Gegenstrom führt, danach in einer Desorptionskolonne aus dem im Wesentlichen aus Acrylsäure, dem Absorptionsmittel und Nebenkomponenten zusammengesetzten Flüssigkeitsablauf der Absorptionskolonne durch Strippen (Abstreifen) mit Inertgas die einfach abtrennbaren leichtflüchtigen Nebenkomponenten weitgehend entfernt und anschließend den (Meth)acrylsäure und das Absorbens als Hauptbestandteile enthaltenden Flüssigkeitsablauf der Desorptionskolonne zur Abtrennung von Roh-(Meth)acrylsäure rektifikativ behandelt.

Alternativ känn man die (Meth)acrylsäure aus den Reaktionsgasen der katalytischen Gasphasenoxidation zunächst in Wasser aufnehmen und anschließend unter Zusatz eines organischen azeotropen Schleppers aus den wässrigen (Meth)acrylsäure enthaltenden Gemischen das Wasser rektifikativ abtrennen.

Die Notwendigkeit einer wirksamen Stabilisierung gegen vorzeitige Polymerisation besteht auch bei der rektifikativen Herstellung von Reinacrylsäure (Reinheit >99,7 Gew.-%) aus Roh-Acrylsäure (Reinheit >99 Gew.-%).

Bei allen vorgenannten Rektifikationsaufgaben ist die erfindungsgemäße Inhibitorzusammensetzung anwendbar. Die erfindungsgemäße Stabilisierung empfiehlt sich auch für den Fall einer kristallisativen Abtrennung von (Meth)acrylsäure oder deren Ester enthaltenden Gemischen.

(Meth)acrolein ist in entsprechender Weise durch katalytische Gasphasenoxidation erhältlich, wobei die Oxidation nur bis zur ersten Oxidationsstufe geführt wird. Das im Reaktionsgasgemisch enthaltene (Meth)acrolein wird in der Regel zunächst mittels Wasser extraktiv aus dem Reaktionsgasgemisch abgetrennt und anschließend destillativ (rektifikativ) aus der wässrigen Lösung gewonnen. Für alle genannten Prozessschritte eignet sich die erfindungsgemäße Inhibitorzusammensetzung.

Die synergistische Wirksamkeit der erfindungsgemäßen Inhibitorzusammensetzung ist im Wesentlichen unabhängig vom pH-Wert. Sie zeigt sich sowohl bei niederen (z. B. Raumtemperatur) als auch erhöhten Temperaturen, wie sie z. B. im Rahmen von thermischen physikalischen Trennverfahren oder chemischen Umsetzungen üblich sind.

In der Regel werden die Gesamtmenge der erfindungsgemäßen Inhibitorzusammensetzung und die relativen Mengen der Komponenten so gewählt, dass sie in der zu stabilisierenden Substanz in ihrer Einsatzmenge in vollem Umfang löslich sind. Häufig erfolgt die Zugabe nicht als Reinsubstanz, sondern als Suspension, Emulsion oder Lösung. Als Lösungs- und/oder Dispergiermedium kommen insbesondere diejenigen Substanzen in Betracht, die Bestandteil des zu stabilisierenden Systems sind, z. B. bei chemischen Umsetzungen, wie Veresterungen, Edukte bzw. Produkte der Umsetzung; bei Extraktionen insbesondere das Extraktionsmedium oder eine Komponente davon.

Besonders geeignete Kombinationen aus N-Oxyl-Radikal a) und Verbindung der allgemeinen Formel (X) sind:

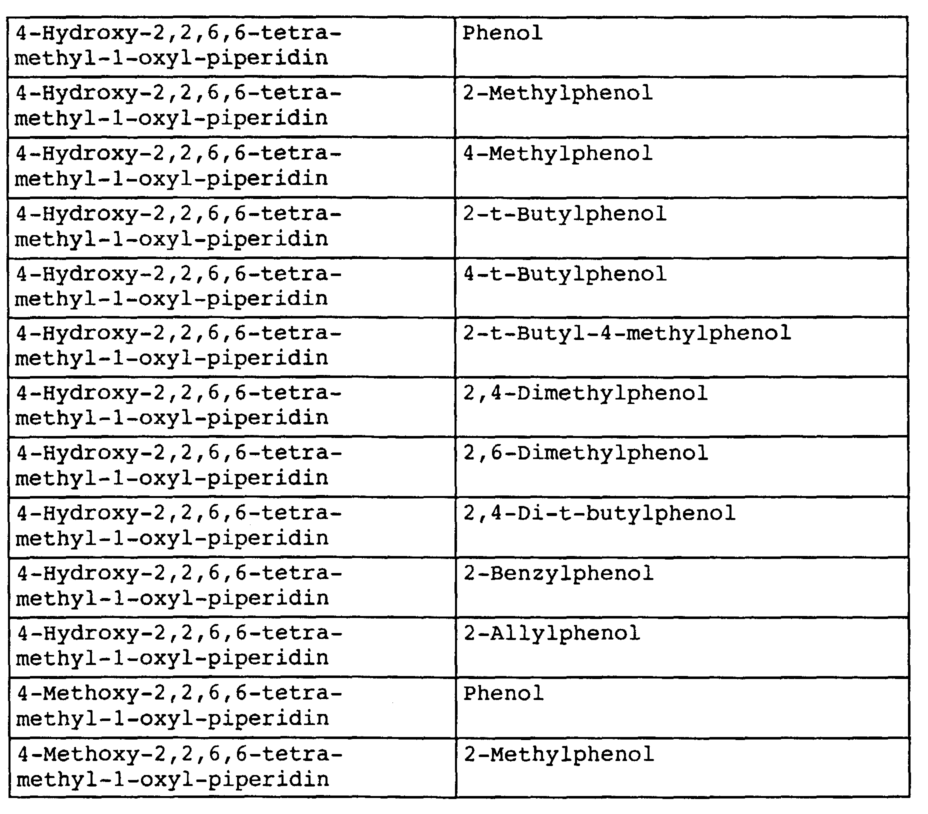

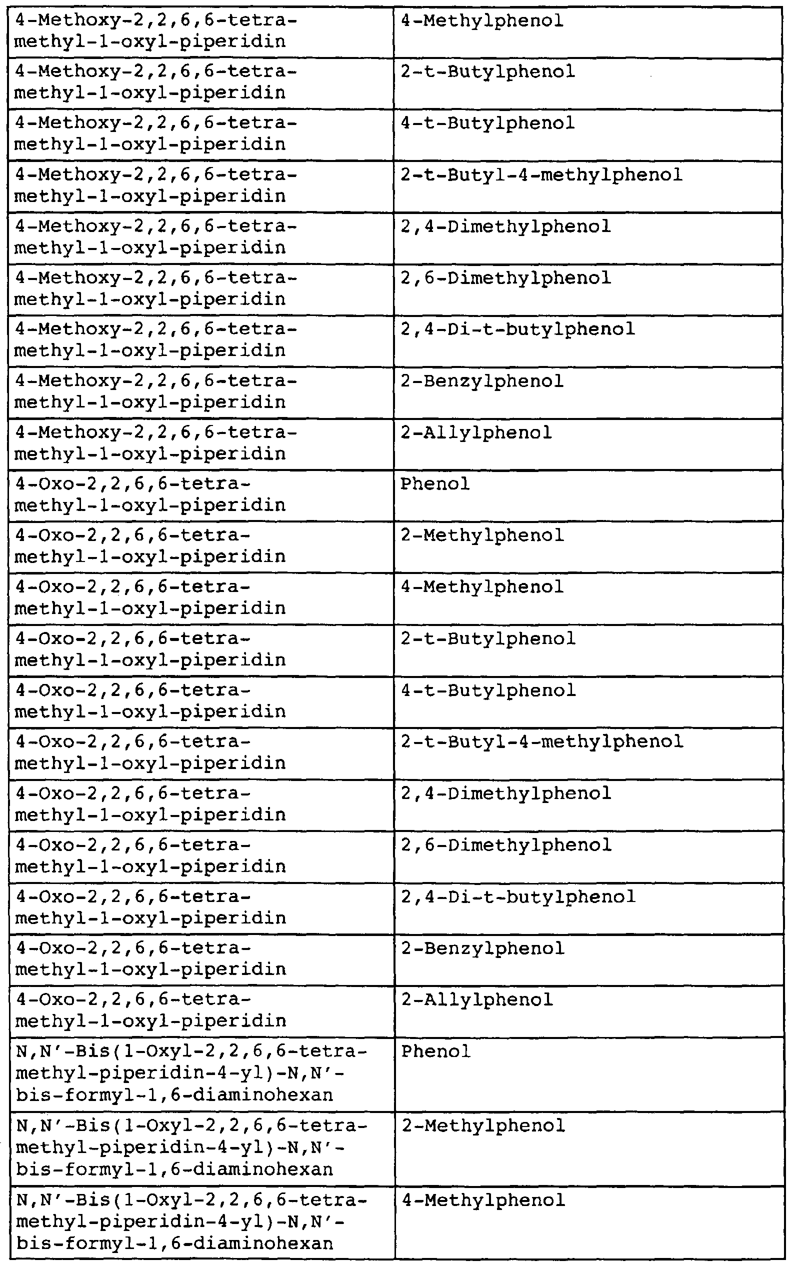

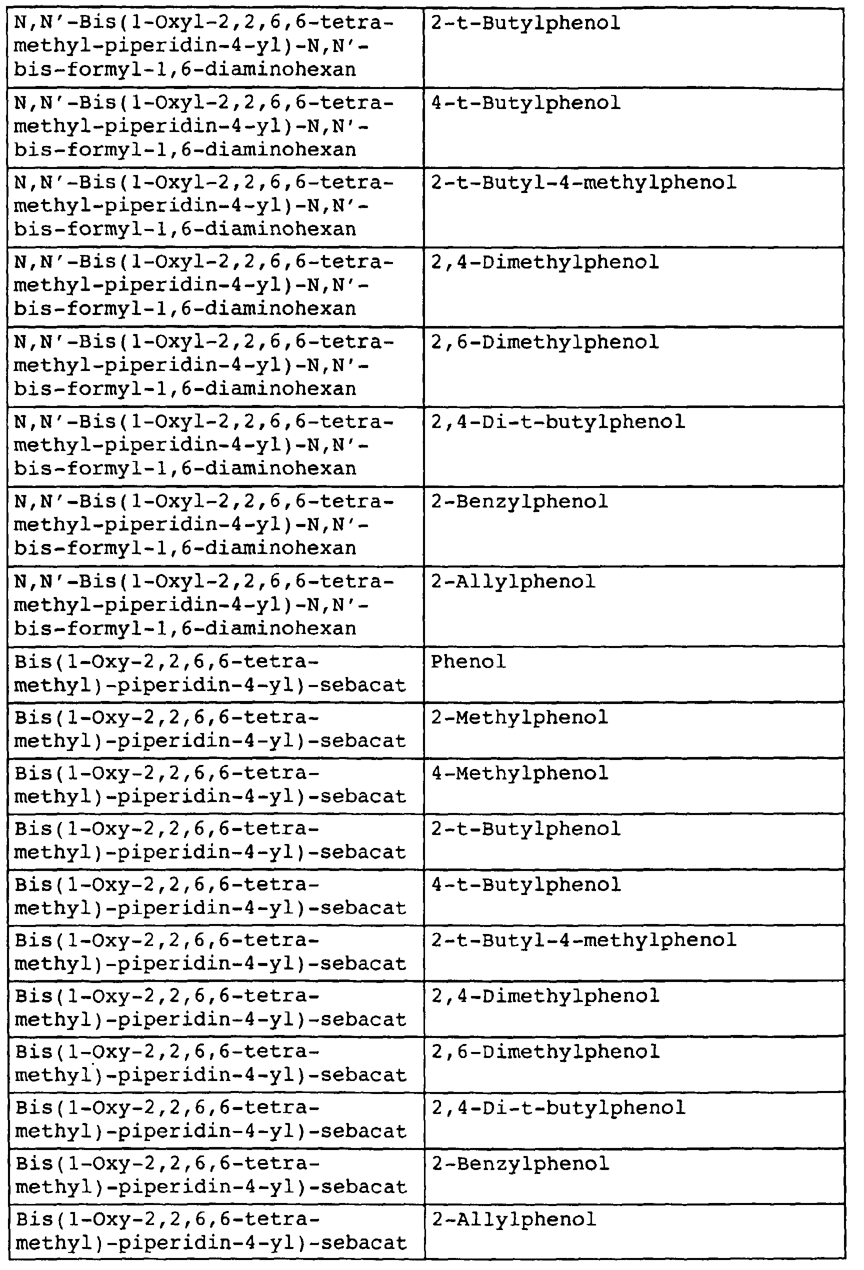

Sie eignen sich insbesondere zur Polymerisationsinhibierung von (Meth)acrolein, (Meth)acrylsäure und deren Estern.

Die Erfindung wird durch die folgenden Beispiele näher veranschaulicht.

### Beispiel 1

2 ml Acrylsäure wurden jeweils mit verschiedenen Polymerisationsinhibitoren versetzt und in einer Glasampulle (20 ml Innenvolumen) luftgesättigt gasdicht eingeschlossen. Dann wurde die Glasampulle in ein 125 °C heißes Ölbad so eingetaucht, dass der Flüssigkeitsspiegel der Acrylsäure und der Flüssigkeitsspiegel des Ölbades sich auf gleicher Höhe befanden. Anschließend wurde die Zeit bestimmt, bis die Acrylsäure auspolymerisiert war (der visuell ermittelte Zeitpunkt, zu dem die Acrylsäure verfestigt war).

Die nachfolgende Tabelle zeigt die erhaltenen Ergebnisse (Mengenangaben in Gew.-ppm, bezogen auf die Acrylsäuremenge).

| | Zeit [min] | |
|---|---|---|
| Verbindung (X) (50 ppm) | ohne Nitroxylverbindung | + 20 ppm Hydroxy-TEMPO* |
| (keine) | | 80 |
| Phenol | 13 | 134 |
| 2-t-Butylphenol | 18 | 122 |
| 2-t-Butyl-4-methyl-phenol | 35 | 190 |
| 2,4-Dimethylphenol | 40 | 242 |
| 2,6-Dimethylphenol | 35 | 225 |
| 4-t-Butylphenol | 18 | 132 |
| 4-Methylphenol | 18 | 129 |
| 2-Benzylphenol | 18 | 109 |
| 2,4-Di-t-Butylphenol | 26 | 124 |
| 2-Allylphenol | 25 | 167 |

| | | |
|---|---|---|
| * 4-Hydroxy-2,2,6,6-tetramethyl-1-oxylpiperidin | | |

### Beispiel 2

Es wurden Stabilitätsprüfungen im "Karussell-Thermostat" durchgeführt. Der "Karussell-Thermostat" besteht aus einem runden Metallgestell zur Aufnahme von Glasampullen und einem mit Heizbadflüssigkeit (Siliconöl) gefüllten Glasbehälter. Die Heizbadflüssigkeit wurde mit einem Tauchsieder auf 120 °C erwärmt. Die Temperatur wurde mit einem Kontaktthermometer geregelt. Das runde Metallgestell hing in der Heizbadflüssigkeit und war mit einer Gewindestange oberhalb des Thermostaten in einem Rührermotor befestigt. Der Rührermotor drehte das Metallgestell langsam. In jeder eingesetzten Glasampulle befand sich ein kleiner Magnetrührstab. Bei jeder Umdrehung des "Karussells" wurden die Ampullen an einem Magneten vorbeigeführt und die Magnetrührstäbe ausgelenkt.

In je einer 50 ml-Glasampulle (mit VE-Wasser gewaschen, getrocknet und 3 Stunden bei 400 °C geglüht) wurden 10 g der zu untersuchenden Probe vorgelegt. Die Proben wurden in den "Karussell-Thermostaten" eingebaut. Gemessen wurde die Zeit bis zur ersten Trübung.

| Inhibitor | Induktionsperiode [Minuten] |
|---|---|
| 10 ppm Hydroxy-TEMPO | 27 |
| 250 ppm 2,4-Di-t-butylphenol | 77 |
| 10 ppm Hydroxy-TEMPO + 250 ppm 2,4-Di-t-butylphenol | 140 |
| 250 ppm 4-Methylphenol | 94 |
| 10 ppm Hydroxy-TEMPO + 250 ppm 4-Methylphenol | 147 |

### Beispiel 3 (die Bezugsziffern beziehen sich auf Fig. 1)

Aus einer heterogen katalysierten Gasphasenoxidation wurde ein eine Temperatur von 270 °C aufweisendes Produktgasgemisch (1) der nachfolgenden Zusammensetzung erhalten.
- 11,5 Gew.-%: Acrylsäure
- 0,3 Gew.-%: Essigsäure
- 30,0 Gew.-ppm: Propionsäure
- 0,09 Gew.-%: Maleinsäureanhydrid
- 0,1 Gew.-%: Acrolein
- 0,1 Gew.-%: Formaldehyd
- 30,0 Gew.-ppm: Furfural
- 0,01 Gew.-%: Benzaldehyd
- 0,3 Gew.-%: Propen
- 3,4 Gew.-%: Sauerstoff
- 5,3 Gew.-%: Wasser
- 1,7 Gew.-%: Kohlenoxide, und als Restmenge N₂

Das Produktgasgemisch (3600 g/h) wurde in einem Sprühkühler (2) auf eine Temperatur von 136 °C abgekühlt. Als Sprühflüssigkeit wurden 750 g/h (7) von insgesamt 7000 g/h über den Fangboden (5) (mit einer Temperatur von 100 °C) aus der Trennkolonne (3) entnommener Schwersiedefraktion (6) verwendet (Sumpfflüssigkeit 4 wurde nicht mitverwendet). Über den mit Wärmeträgeröl betriebenen Rohrbündelwärmetauscher (8) wurde die Sprühflüssigkeit im Kreis geführt. 40 g/h Schwersieder wurden dem Kreislauf kontinuierlich entnommen.

Das auf eine Temperatur von 136 °C abgekühlte Produktgasgemisch wurde unterhalb des Fangbodens (5) der Trennkolonne zugeführt (10).

Die Kolonne war eine Bodenkolonne mit 25 Dual-flow- und 50 Glokkenböden. Der Boden oberhalb von Boden 15 war als weiterer Fangboden (11) gestaltet. Über ihm wurden 1800 g/h einer eine Temperatur von 97 °C aufweisenden Rohacrylsäure (12) mit einem Acrylsäuregehalt von 97,3 Gew.-% abgezogen und einem Suspensionskristallisator (13) zugeführt. Ein Teil (6250 g/h) der entnommenen Schwersiederfraktion (14) wurde in einem mit Wärmeträgeröl betriebenen Rohrbündelwärmetauscher (15) auf 105 °C erwärmt und auf den 5. Boden in die Kolonne rückgeführt (16).

Die bei der Kristallation erzeugte Suspension (Feststoffgehalt ca. 25 Gew.-%) wurde auf einer Zentrifuge diskontinuierlich in Kristalle und Mutterlauge getrennt. Die Mutterlauge (1255 g/h) wurde auf den 15. Boden in die Trennkolonne rückgeführt (28).

Die Analyse der Kristalle ergab einen Acrylsäuregehalt von 99,5 Gew.-%.

Am Kopf der Kolonne wurde ein gasförmiger Strom (17) entnommen und im Sprühkühler (18) einer Partialkondensation unterworfen. 480 g/h des dabei anfallenden Kondensates (Sauerwasser) wurde am Kolonnenkopf mit einer Temperatur von 30 °C in die Kolonne zurückgeführt (26). 170 g/h des Sauerwassers wurden kontinuierlich entnommen. 40 g/h des entnommenen Sauerwassers wurden mit OH-TEMPO (22) versetzt und als 0,5 gew.-%ige wässrige Stabilisatorlösung (21) gemeinsam mit der Restmenge des Sauerwassers (23) über den wassergekühlten Rohrbündelwärmetauscher (24) auf 18 °C abgekühlt als Sprühflüssigkeit (25) verwendet. Mit einem Teil der Mutterlauge wurde eine Lösung mit 2,0 gew.-%igem 2,4-Di-t-butylphenol (DTBP) hergestellt, die in einer Menge von 25 g/h auf dem 37. Boden der Trennkolonne zugeführt wurde (27).

Die beschriebene Trennvorrichtung konnte über 360 h ohne nennenswerte Polymerisatbildung betrieben werden.

Wurde auf die Stabilisatorzufuhr auf dem 37. Boden (27) verzichtet, kam es auf den unteren Glockenböden innerhalb von 70 Stunden zu starker Polymerbildung und Verstopfungen.

## Patentansprüche

1. Phosphorfreie Inhibitorzusammensetzung, umfassend
a) wenigstens ein N-Oxyl-Radikal und
b) eine Verbindung der allgemeinen Formel (X) 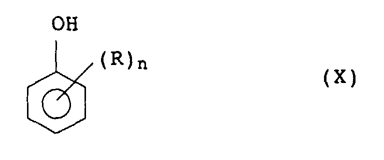 worin
n für eine ganze Zahl von 0 bis 3 steht und
R für C₁-C₄-Alkyl, C₂-C₄-Alkenyl oder C₇-C₁₀-Aralkyl steht.

2. Zusammensetzung nach Anspruch 1, wobei die Verbindung der Formel (X) unter Phenol, 2-Methylphenol, 4-Methylphenol, 2-t-Butylphenol, 4-t-Butylphenol, 2-t-Butyl-4-methylphenol, 2,4-Dimethylphenol, 2,6-Dimethylphenol, 2,4-Di-t-butylphenol, 2-Benzylphenol oder 2-Allylphenol oder Mischungen davon ausgewählt ist.

3. Zusammensetzung nach Anspruch 1 oder 2, wobei das N-Oxyl-Radikal ein Piperidin-, Pyrazolidin- und/oder Pyrrolidin-N-oxylradikal ist.

4. Zusammensetzung nach Anspruch 3, wobei es sich bei dem N-Oxyl-Radikal um 4-Hydroxy-2,2,6,6-tetramethyl-1-oxyl-piperidin handelt.

5. Verwendung der Inhibitorzusammensetzung nach einem der Ansprüche 1 bis 4 zur Verhütung unerwünschter Polymerisation von ethylenisch ungesättigten Verbindungen.

6. Verfahren zur Stabilisierung von Materialien, die wenigstens eine ethylenisch ungesättigte Verbindung umfassen, gegen unerwünschte Polymerisation, indem man zu den zu stabilisierenden Materialien eine Inhibitorzusammensetzung nach einem der Ansprüche 1 bis 4 gibt.

7. Stoffgemisch, enthaltend wenigstens eine ethylenisch ungesättigte Verbindung und eine zur Verhütung unerwünschter Polymerisation wirksame Menge der Inhibitorzusammensetzung nach einem der Ansprüche 1 bis 4.
